**Europäisches Patentamt**

**European Patent Office**

**Office européen des brevets**

(19)

(11) Publication number: **0 131 660**
**B1**

(12)    # EUROPEAN PATENT SPECIFICATION

(45) Date of publication of patent specification: **23.11.88**

(51) Int. Cl.⁴: **G 01 T 1/164**

(21) Application number: **83304057.9**

(22) Date of filing: **13.07.83**

(54) Radioactivity detection system.

(43) Date of publication of application:
**23.01.85 Bulletin 85/04**

(45) Publication of the grant of the patent:
**23.11.88 Bulletin 88/47**

(84) Designated Contracting States:
**DE GB**

(56) References cited:
**FR-A-2 237 206**
**US-A-3 594 577**
**US-A-4 267 452**

**IEEE NUCLEAR SCIENCE NS-29 (1982-02) no. 1,
NEW YORK (US), K. TAKAMI et al.: "Design
consideration for a continuously rotating
positron computed tomograph", pp. 534-538**

(73) Proprietor: **Scinticor Inc.**
**7865 N. 86th Street**
**Milwaukee WI 53224 (US)**

(72) Inventor: **Govaert, Johan A.**
**One Green Street**
**Peabody Massachusetts 01960 (US)**
Inventor: **Town, Joseph E.**
**111 Russell Street**
**Peabody Massachusetts 01960 (US)**

(74) Representative: **Sunderland, James Harry et al
HASELTINE LAKE & CO Hazlitt House 28
Southampton Buildings Chancery Lane
London WC2A 1AT (GB)**

Courier Press, Leamington Spa, England.

EP 0 131 660 B1

## Description

The present invention relates generally to detecting systems and, more particularly, to a radioactivity detection system and detectors therefor.

Various types of radioactivity distribution measurement systems are known in the art for determining the location of radioactive material injected in diagnostic amounts into a human body or the like. These systems utilize an array of scintillators for sensing radioactive events within the body and a plurality of photomultiplier tubes that are coupled to the scintillators for detecting the sensed events. The address location for each activated scintillator is linearly encoded and then processed to provide a pictorial representation of the relative radioactive event levels detected by the scintillators. Spatial resolution is limited by the smallest diameter of available photomultiplier tubes.

Such systems have suffered from the disadvantages that linear encoding of scintillator address locations requires an excessive number of photomultiplier tubes in order to provide each scintillator with a unique address location. A large number of photomultiplier tubes increases the system costs and increases the likelihood for system downtime. Such systems also suffer from the disadvantage that the dark current of the photomultiplier tubes causes pulses (dark noise) which, in certain instances, are incorrectly interpreted as valid scintillations.

U.S. Patent 3 594 577 discloses a system in which a plurality of optically screened scintillator elements are arranged in a row and column matrix. Light detectors are also arranged in rows and columns, more widely spaced than the scintillator elements, each with a "field of view" extending over a plurality of scintillator elements and overlapping the field of view of neighboring detectors.

IEEE Transactors on Nuclear Science, Vol. NS-29 (1982) Febr., No. 1, K. Takami et al, pages 534 to 538, "Design consideration for a continuously rotating positron computed tomograph", concerns a whole body, multisclice positron computed tomograph having three detector rings with 160 so-called BGO crystals per ring. Around each ring so-called BGO quad-detectors are provided. Each detector has four crystals attached together with the two outer crystals optically isolated from the inner crystals. Two detectors are mounted on the four crystal, each detector juxtaposed to one outer and one inner crystal.

In U.S. Patent 4 267 452 a system is disclosed in which three photomultiplier tubes are required to determine the position of an activated scintillator. This system shows one photomultiplier tube superposed on four crystal assemblies, with each assembly containing four scintillators. There are thus sixteen scintillators that potentially can provide light signals, in various amounts, to one photomultiplier tube. This in turn requires a rather complex anticoincidence/coincidence logic in the processing of the signals from the photomultiplier tubes.

An embodiment of the present invention can overcome the above shortcomings by providing a radioactivity detection system for presentation of radioactive event distributions.

According to the present invention there is provided a radiation detection assembly, comprising

an array of scintillator bodies, each providing a plurality of optically linked scintillators, and

an array of light detectors each arranged for detecting light from four different scintillators provided by four different scintillator bodies, characterised in that

each scintillator body provides only two optically linked scintillators, and in that

with each detector so arranged for detecting light from four different scintillators provided by four different scintillator bodies, light from the other four scintillators belonging to those four scintillator bodies is detected by four different detectors.

In a system using such an assembly, means are provided for mounting the assembly for relative motion to a subject under diagnosis, and processing means coupled to the detector. Preferably, the mounting means is operatively connected to an exercising means for performing physiologic stress analysis of the subject. Preferably, the mounting means is secured on a movable platform, which is connected to the exercising means by a quick connect-disconnect mechanism. This allows the detection system to be moved expeditiously from the stress analysis area to another location, such as a patient's bedside. In a further embodiment, a detector assembly is designed for rotation about the subject.

The resultant radioactivity detection system is a versatile yet compact, high resolution system, which provides more signal output per each light detector than heretofore attainable and it also allows for simpler processing circuitry than heretofore possible.

The invention accordingly comprises the radioactivity detection system of the present disclosure, its components, parts and their interrelationships, the scope of which will be indicated in the appended claims.

Reference is made, by way of example, to the accompanying drawings, wherein:

Fig. 1 is a perspective view of one preferred embodiment of a radioactivity detection system constructed in accordance with the present invention;

Fig. 2 is a perspective view of another preferred embodiment of a radioactivity detection system constructed in accordance with the present invention;

Fig. 3 is a schematic plan view illustrating a dual detection system;

Fig. 4 is a schematic cross section, illustrating another preferred embodiment of the present invention;

Fig. 5 is a block and schematic diagram of still another preferred embodiment of a radioactivity detection system constructed in accordance with the present invention;

Fig. 6 is a perspective view of a crystal scintillation detector assembly of the radioactivity detection systems illustrated above;

Fig. 7 is a schematic diagram showing one preferred positional relationship between a plurality of crystal scintillator assemblies and a plurality of light detectors;

Fig. 8 is a vertical section along the line 8—8 of Fig. 7;

Fig. 9 is a schematic diagram showing another preferred positional relationship between a plurality of crystal scintillator assemblies and a plurality of light detectors;

Fig. 10 is a vertical section along the line 10—10 of Fig. 9;

Fig. 11 is a schematic diagram similar to the one shown in Fig. 7 but illustrating a different embodiment of the present invention;

Fig. 12 is a vertical section along the line 12—12 of Fig. 11; and

Fig. 13 is a graph illustrating certain principles of the present invention.

In general, a radioactivity detection system 10 is used for determining the location and distribution of radioactive events emitted from a subject under diagnosis into which a diagnostic amount of radioactive material has been injected.

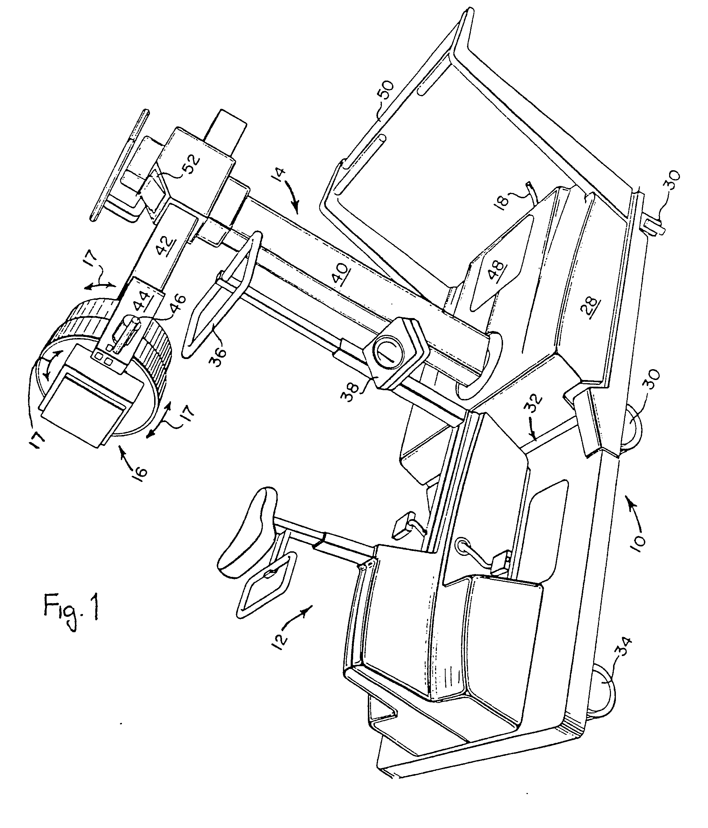

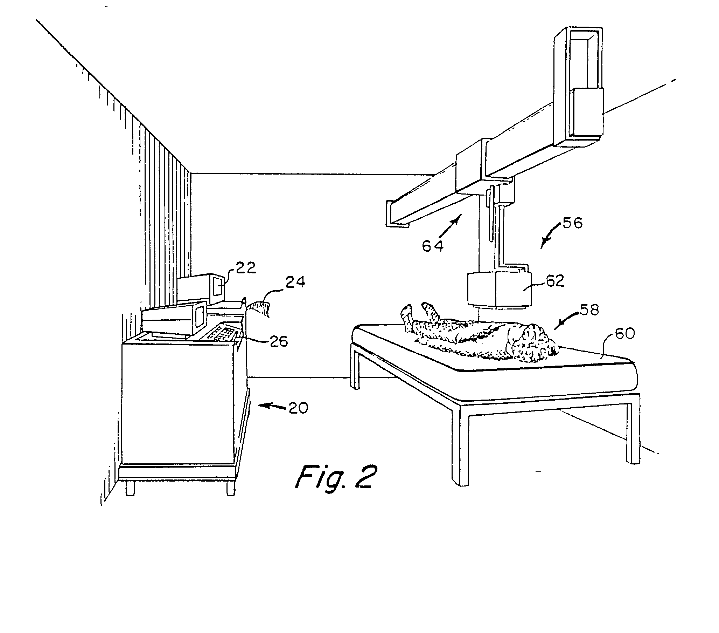

A perspective view of one preferred embodiment of such a radioactivity detection system 10 is illustrated in Fig. 1. System 10 is a high resolution system specifically designed for physiologic stress analysis, such as heart function, of the subject, not shown. The radioactivity detection system 10 essentially comprises an exercising means 12, a mounting means 14 and a radioactivity distribution detector 16 secured on the mounting means 14. By means of a cable 18, the radioactivity distribution detector 16 is designed to be connected to a suitable processing means 20, such as the one illustrated in Fig. 2. The processing means 20 preferably includes a visual display 22, a graphic recorder 24, and a manual data input terminal 26, such as a keyboard. The exercising means 12 may comprise an exercise bike as shown, or a treadmill, not shown. Preferably, the mounting means 14 is secured on a platform 28 supported on casters 30. In turn, the platform 28 is connected to the exercising means 12 via a suitable quick connect-disconnect mechanism 32, which may take the form of a pair of arms projecting from the exercising means 12 and designed to be received within appropriate depressions formed in the platform 28. The exercising means 12 preferably also is mounted on a pair of wheels 34 so as to be on the same approximate height above the floor as the platform 28. The radioactivity detection system 10 thus can be used for stress analysis of an ambulatory subject, or it can be used for bedside analysis of a bedridden patent.

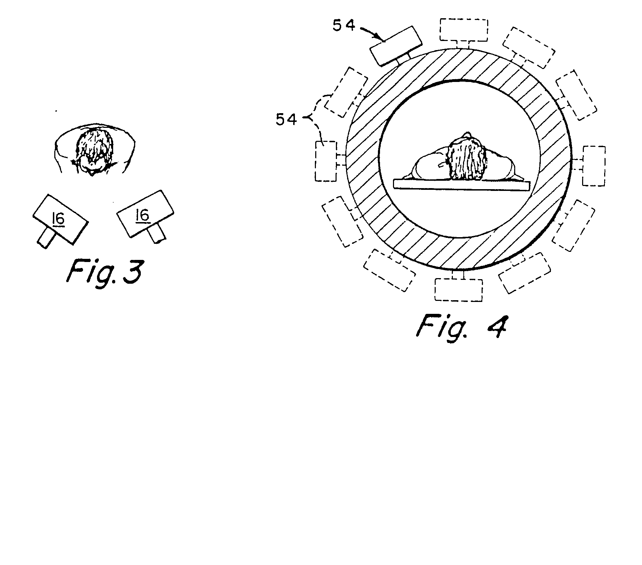

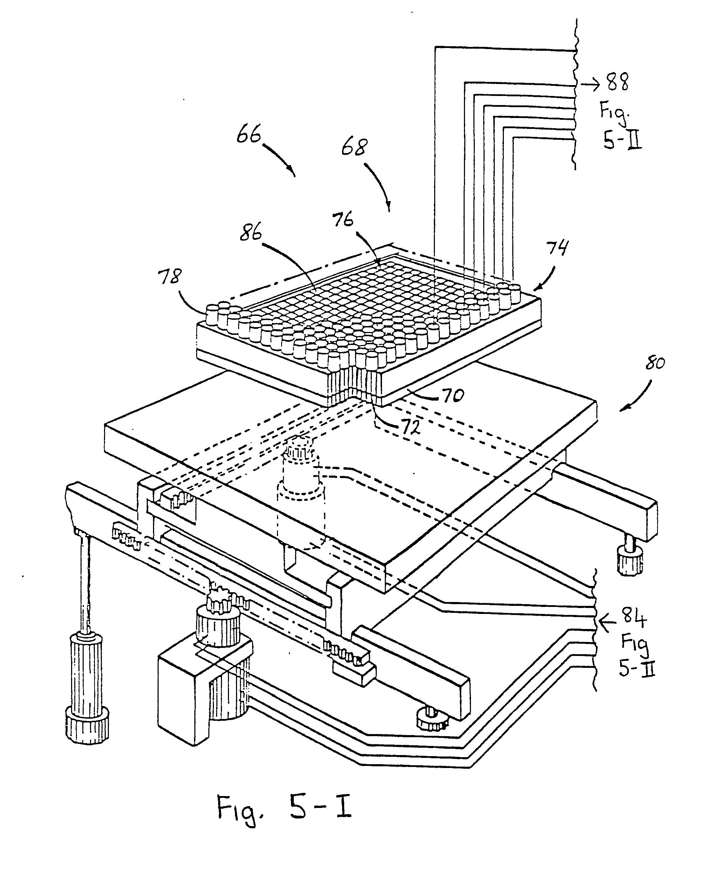

The exercising means 12 preferably is provided with a handle bar 36, whose height is adjustable, and a display 38, indicating the level of exertion by the subject. The exercising means 12 preferably rotates about a fixed vertical axis coincident with that of the subject's heart, so that rotation of the means 12 does not cause lateral displacement of the heart out of the detector's 16 field of view. The detector mounting means 14 preferably is three-dimensionally adjustable so as to allow for the desired positioning of the radioactivity distribution detector 16 with respect to the subject, whether the subject is exercising on the exercising means 12 or is bedridden. To this end, the mounting means 14 preferably comprises a rotatable vertical post 40, carrying a horizontal member 42 for translational motion with respect thereto. At its forward end, the member 42 has journalled thereto a yoke 44, by means of which the detector 16 can be adjusted angularly in all directions, as shown by the arrows 17. Positioning of the detector 16 is effected by a handle 46. When the mounting means 14 is in transport or storage, the detector 16 preferably is made to rest at an area 48 on the platform 28. The platform 28 furthermore is provided with a horizontal bar 50, with which it may be pushed or pulled to a location, as desired. A further display 52 preferably is carried by the mounting means 14 so as to indicate, to the subject and attendant alike, the level of radioactivity being detected. The three-dimensional adjustability of the mounting means 14 can be effected either manually, pneumatically or electrically by known means, not otherwise disclosed and built into the platform 28. Further, and as illustrated in Fig. 3, the radioactivity detection system 10 also can include two such radioactivity distribution detectors 16 so as to allow analysis of the same event from two detectors 16 viewing the subject with an intersecting angle. Still further, and as illustrated in Fig. 4, a radioactivity detector assembly 54 also can be mounted for rotation about the subject, or in the alternative, a plurality of such detector assemblies 54, shown in dotted lines, can be mounted circumferentially about the subject.

Another preferred embodiment of a radioactivity detection system 56 is shown in Fig. 2. System 56 is designed for detection of radioactive events in a subject 58 being supine on a table 60, or the like. A radioactivity distribution detector 62 is suspended from a mounting means 64 designed for effecting relative motion between the detector 62 and the subject 58. Such relative motion, both vertical and horizontal, preferably is effected by a suitable electric motor, not shown. Other means, e.g., pneumatic and/or hydraulic, can be employed equally. The radioactivity detection system 56 otherwise is identical with the system 10 shown in and described with reference to Fig. 1 and, as such, fully interfaces with the processing means 20 above described.

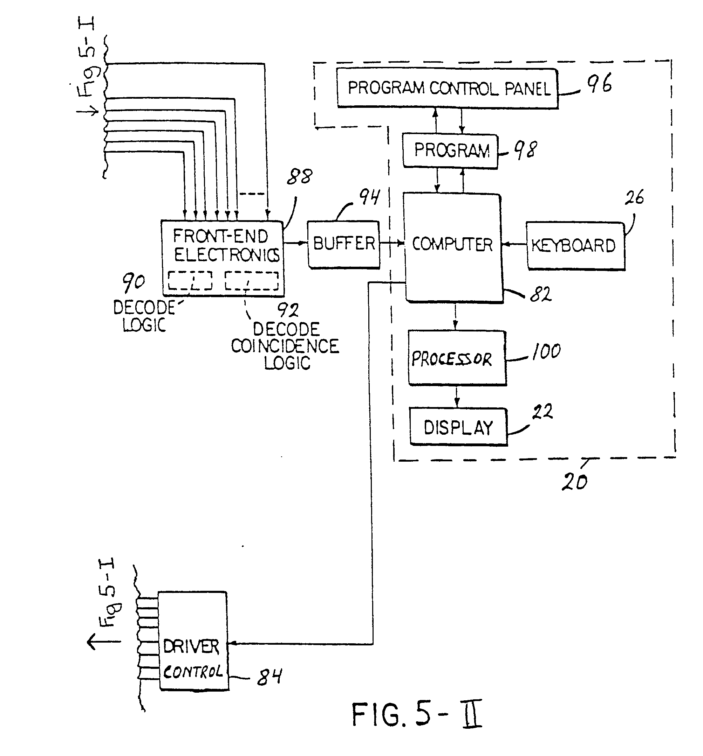

Still another preferred embodiment of a radioactivity detection system 66 is shown in Fig. 5. System 66 comprises a detector assembly 68 which includes a collimator 70 with collimator bores 72, an array 74 of scintillation crystal assemblies 76 and a plurality of superposed light detectors, such as photomultiplier (PM) tubes 78. A subject (not shown) under diagnosis is positioned on a programmable XY platform 80 which is in spaced relation to detector assembly 68, with a portion of the subject being in registration with collimator 70. Responsive to command signals generated by a computer 82 in the processing means 20, scanning signals are generated by a driver control 84 which operates to move the platform 80 in a specified scanning pattern. Conversely, another preferred embodiment would have the detector assembly 68 moving on a programmable XY platform, while the patient remains stationary. A scintillator 86, having a unique address location in the array 74 and being in registration with one collimator bore 72, when activated by securing radioactivity, emits a light signal detected by two adjacent superposed detectors 78. Data signals generated by the detectors 78 first are processed in front-end electronics which includes a decode logic 90 and a decode coincidence logic 92. The decode coincidence logic 92 ensures that data signals have been detected from two, and only two, adjacent detectors 78 which define a unique address for the scintillation event. Events sensed at each unique address are accumulated in a buffer memory 94. Upon completion of each scanning step, the events stored in the buffer memory 94 are fed to the computer 82 in the processing means 20 and the memory 94 cleared for reception of new data. Operation of the radioactivity detection system of whichever abovedescribed embodiment is directed from a control panel 96. Panel 96 preferably is a series of switching devices coupled to the computer 82 via a computer interface 98. Any pertinent data conveniently is logged into the display 22 via the keyboard 26. The data in computer 82 preferably is fed to a microprocessor driven display processor 100 which encodes the scintigraphic data into Gray shade and/or color television signals which drive the visual display 22, which preferably is a raster scan display.

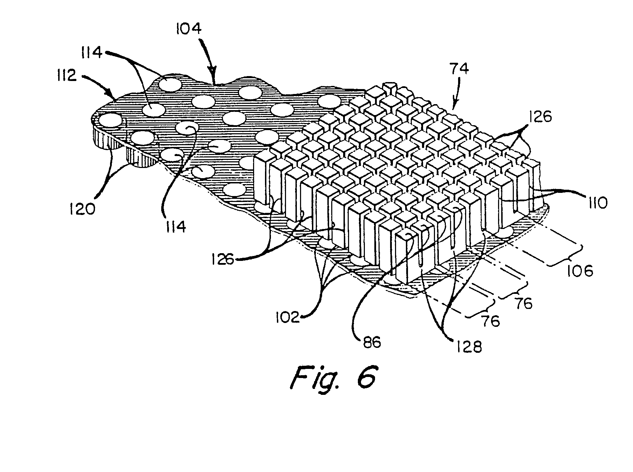

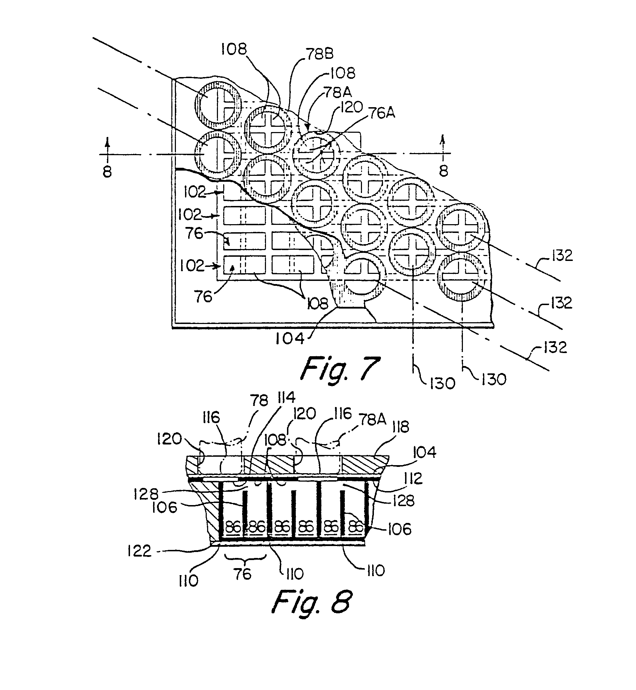

One preferred crystal detector assembly 68 of the radioactivity detection system of whichever above-described embodiment is shown in perspective in Fig. 6. The crystal detector array 74 of assembly 68 comprises a plurality of the scintillation crystal assemblies 76 mounted in parallel rows 102 onto a glass plate 104. Each crystal assembly 76 is formed of a unitary body having a single slot 106 that extends inwardly from one end of the body to define two sensing heads, i.e., scintilators 86, 86. Preferably, each crystal assembly 76 is composed of a scintillation material. Such scintillation material preferably comprises a thallium activated sodium iodide crystal —NaI(Tl), or a cesium iodide crystal —CsI, or a bismuth germanate crystal —$Bi_4 Ge_3 O_{12}$, or cadmium tungstate, or the like. The ends of each crystal assembly 76 opposite to the scintillators 86, 86 form two exit windows 108, 108, one each in registration with a corresponding one of the scintillators 86, 86, observe Fig. 8. The slot 106, as well as the openings 110 in between adjacent crystal assemblies 76, are filled with a reflective material, for example magnesium oxide. Further, the glass plate 104 also preferably is selectively coated, as at 112, with a reflective material, except for those areas, as at 114, corresponding to the active internal diameter areas 116 of the PM tubes 78 shown in Fig. 8. Preferably, a plate 118, which may be formed of aluminum or the like, is added to the assembly 68 and glued to the glass plate 104 on the side facing the PM tubes 78. The primary function of this plate 118 is to add structural strength and rigidity to the assembly 68 and to provide proper and accurate positioning of the PM tubes 78 within appropriate locating holes 120. It will be observed in Fig. 8 that these locating holes 120 are formed with diameters slightly larger than the outside diameters of the PM tubes 78 so that the tubes 78 may fit snugly therein. A cover plate 122, added on the radiation entrance side, completes the crystal detector assembly 68 and serves as the radiation entrance window thereof. Preferably, the cover plate 122 also is formed of a material with a minimum radiation attenuation coefficient for gamma rays of 50 keV and greater, e.g., aluminum.

The array 74 of crystal assemblies 76 may be made in any convenient manner. For example, a slab of scintillator material first is prepared to the required dimensions. Next, a series of equidistantly placed parallel channels 126 are cut in the slab, preferably all the way through so as to form the parallel rows 102. Then, in a direction normal to the direction of the channels 126, the slots 106 and the openings 110 are alternately cut in the slab so as to form the plurality of crystal assemblies 76. It will be noted that the openings 110 preferably are cut all the way through, but not the alternate slots 106, so as to leave a bridge 128 of scintillator material preferably adjacent the exit windows 108, 108 in each crystal assembly 76. The significance of this bridge 128 and its thickness will be more fully described below. Thereafter, the slots 106, the openings 110 and the channels 126 preferably are filled with the reflective material, and the cover plate 122 secured in place.

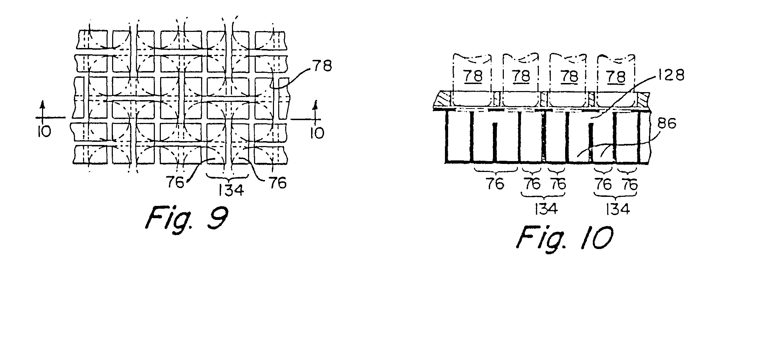

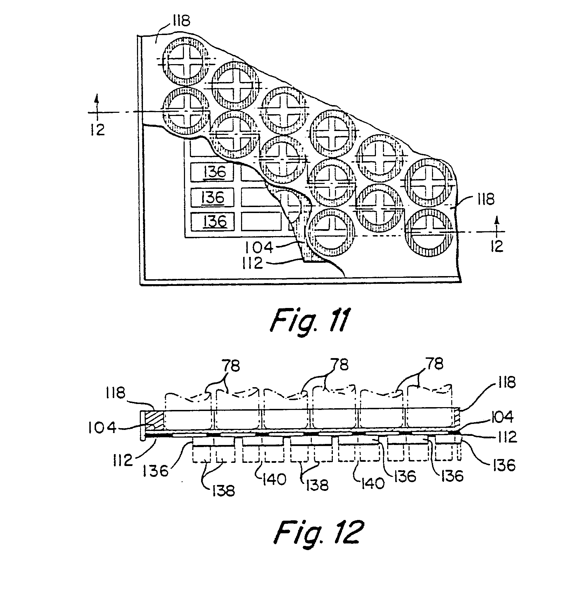

One preferred positional relationship between a plurality of crystal assemblies 76 and a plurality of light detectors 78 of the crystal detector assembly 68 is shown schematically in Fig. 7. Other preferred positional relationships between the crystal assemblies and the light detectors are shown, respectively, in Figs. 9 and 11. The underlying principle, which we call the doublet principle, however is the same for each of these illustrated preferred positional relationships. This doublet principle provides the radioactivity detection system of the invention in each of the above-described embodiments with a simple coding scheme. According to this coding scheme, light generated by a scintillation in one scintillator 86 of one crystal assembly 76 is designed to fall on, and thus activate, two adjacent light detectors, e.g., PM tubes 78, but with one PM tube 78 receiving more light than the other.

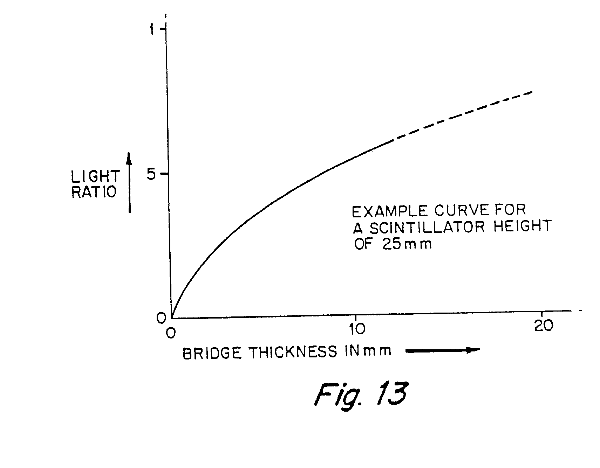

As will be evident from viewing the respective drawings, one PM tube 78A is associated with four crystal assemblies 76A, with each assembly 76 comprised of two scintillators 86, 86, that is eight scintillator crystals altogether. Of these eight scintillators 86, four scintillators 86 are directly underneath the PM tube 78A and four scintillators 86 are moved over one position. These four, one position removed scintillators 86, are respectively associated with four adjacent PM tubes 78. A scintillation occurring in one of the four scintillators 86 directly underneath the PM tube 78A casts more light on that PM tube 78A than on the adjacent PM tube 78B, and it causes no light to be cast on any other PM tube 78. The emission of a lesser amount of light from the scintillator 86 found directly underneath the PM tube 78A on the adjacent PM tube 78B is effected by the bridge 128 of scintillation material, which in fact physically connects and optically couples the two adjacent scintillators 86, 86 into the one crystal assembly 76, observe Fig. 8. The thickness of this bridge 128 above the slot 106 relative to the height of the crystal scintillators 86, 86 governs the light distribution between the two adjacent PM tubes 78A and 78B. The ratio of bridge 128 thickness to scintillator 86 height must always be greater than zero. In Fig. 13, there is illustrated the variation of the light distribution with the thickness of the bridge 128, with the bridge 128 functioning as a light mixer. Fig. 13 plots the light ratio versus the thickness of the bridge 128. The light ratio is defined for the example PM tubes 78A and 78B as the amount of light received by 78B divided by the amount of light received by tube 78A. As may be observed therein, the light ratio always should be less than one under all conditions, keeping in mind statistical fluctuations. The light ratio must be large enough, however, to assure that the electrical signals from both PM tubes 78A and 78B are sufficiently high so as to exceed anticipated noise levels in background or in the processing circuitry.

The preferred positional relationship as depicted in Fig. 7 is achieved by arranging the PM tubes 78 in parallel rows 130 running in a direction normal to the parallel rows 102 of the crystal assemblies 76, and in further parallel rows 132 running at an angle to both the row 130 and the rows 102.

The second preferred positional relationship between a plurality of crystal assemblies 76 and a plurality of light detectors 78 is depicted in Figs. 9 and 10. In this arrangement, preferably a pair of crystal assemblies 76 are mounted in one can 134, with adjacent cans 134 disposed normal to one another. The PM tubes 78, on the other hand, are disposed in parallel rows in two directions normal to one another.

In Figs. 11 and 12, there is illustrated a still further embodiment of the present invention. The positional arrangement is similar to that shown in Fig. 7, but in lieu of the crystal assemblies 76, a plurality of wafers 136 are used. The wafers 136 also are formed of a scintillation material, and they may optionally also include a pair of scintillators 138, 138. If so, the slot 140 between the scintillators 138, 138 preferably also is filled with a reflective material, not shown. The height of the scintillators 138 can vary from a theoretical zero to some positive value, although it would always be somewhat less than the maximum height of the scintillators 86. The embodiment shown in Figs. 11 and 12 particularly is useful for low energy isotopes which characteristically require less scintillation material so as to generate a photon therein. This embodiment also incorporates the doublet principle above mentioned in that the light of a scintillation in half of one wafer 136 is detected by two adjacent PM tubes 78, with one detector receiving more light than the other. The front-end electronics 88 will register the corresponding electrical signals generated by the two adjacent PM tubes 78 only if one signal is greater than the other and will reject all other signals as invalid.

Thus it has been shown and described an improved high resolution radioactivity detection system 10 designed for presentation of radioactive event distributions, which system 10 satisfies the objects and advantages set forth above.

## Claims

1. A radiation detection assembly, comprising an array of scintillator bodies (76, 136), each providing a plurality of optically linked scintillators (86), and an array of light detectors (78), each arranged for detecting light from four different scintillators (86) provided by four different scintillator bodies (76, 136), characterised in that each scintillator body (76, 136) provides only two optically linked scintillators (86), and in that with each detector (78) so arranged for detecting light from four different scintillators (86) provided by four different scintillator bodies (76, 136), light from the other four scintillators (86) belonging to those four scintillator bodies (76, 136) is detected by four different detectors (78).

2. An assembly as claimed in claim 1, wherein light arising from a radiation activated scintillator (86) of a scintillator body (76) activates the detector (78) arranged for detecting light from that scintillator more than the detector arranged for detecting light from the other, optically linked, scintillator of the scintillator body.

3. An assembly as claimed in claim 2, wherein each scintillator body (76) is unitary, with a slot (106) extending inwardly from one end of the body to form the two optically linked scintillators (86).

4. An assembly as claimed in claim 3, wherein the light distribution between the two light detectors arranged for detecting light from the two scintillators of a scintillator body is inversely proportional to the extension of the slot (106) into the unitary scintillator body (76).

5. An assembly as claimed in claim 3 or 4, wherein a reflective material is disposed in the slot (106) of each of unitary scintillator body (76).

6. An assembly as claimed in any preceding claim, wherein the scintillators are formed of crystals of cesium iodide, or thallium activated sodium iodide, or bismuth germanate crystals, or cadmium tungstate, and the light detectors are photomultiplier tubes.

7. An assembly as claimed in any preceding claim, wherein the scintillator bodies (76, 136) are arranged, all aligned in one direction, in rows and columns, and the detectors (78) are arranged each to detect light from two scintillators, of neighbouring scintillator bodies, in each of two adjacent rows.

8. An assembly as claimed in any one of claims 1 to 6, wherein the scintillator bodies (76, 136) are mounted in side-by-side pairs, the pairs being arranged in rows and columns with each pair aligned perpendicularly to its neighbouring pairs in the same row and in the same column, and the detectors are arranged in a plurality of parallel rows each for detecting light from one scintillator of each of four different pairs.

9. A radioactivity distribution detection system comprising:
(a) a detector assembly (62, 54) as claimed in any preceding claim;
(b) means (64) for mounting the detector assembly relative to a subject (58) to be analyzed;
(c) means (64) for effecting relative motion between the detector assembly (62, 54) and the subject (58); and
(d) processing means (20) operatively coupled to said detector assembly (62, 54) and including display means (22) for processing and presenting a representation of radioactivity distribution within the subject (58).

10. A radioactivity distribution detection system as claimed in claim 9, wherein the relative motion between the mounting means and the subject is in a circular path about the subject.

11. A radioactivity distribution detection system for stress analysis of a subject comprising:
(a) an exercising means (12);
(b) mounting means (14) operatively associated with said exercising means (12); and
(c) a radiation detection assembly (16) as claimed in any one of claims 1 to 8 secured on said mounting means (14).

12. A radioactivity distribution detection system as claimed in claim 11, wherein said exercising means (12) is a treadmill.

13. A radioactivity distribution detection system as claimed in claim 11, wherein said exercising means (12) is an exercise bike.

14. A radioactivity distribution detection system as claimed in claim 11, 12 or 13, wherein said mounting means (14) is three-dimensionally adjustable.

**Patentansprüche**

1. Strahlungserfassungs-Aufbau, mit

—einem Feld von Szintillator-Körpern (76, 136), wovon jeder mit einer Vielzahl von optisch gekoppelten Szintillatoren (86) versehen ist, und
—einem Feld von Licht-Detektoren (78), wovon jeder zum Erfassen von Licht aus vier verschiedenen Szintillatoren (86) angeordnet ist, die durch vier verschiedene Szintillator-Körper (76, 136) gegeben sind, dadurch gekennzeichnet,
—daß jeder Szintillator-Körper (76, 136) mit nur zwei optisch gekoppelten Szintillatoren (86) versehen ist und
—daß jeder Detektor (78) zum Erfassen von Licht aus vier verschiedenen Szintillatoren (86) angeordnet ist, die durch vier verschiedene Szintillator-Körper (76, 136) gegeben sind, wobei Licht der weiteren vier Szintillatoren (86), die zu diesen vier Szintillator-Körpern (76, 136) gehören, durch vier verschiedene Detektoren (76) erfaßt wird.

2. Aufbau nach Anspruch 1, bei dem Licht, das sich aus einem durch Strahlung aktivierten Szintillator (86) eines Szintillator-Körpers (76) ergibt, denjenigen Detektor (78), der zum Erfassen von Licht aus diesem Szintillator angeordnet ist, stärker als den Detektor aktiviert, der zum Erfassen von Licht aus dem weiteren, optisch gekoppelten Szintillator des Szintillator-Körpers angeordnet ist.

3. Aufbau nach Anspruch 2, bei dem jeder Szintillator (76) einheitlich gestaltet ist, und zwar mittels eines Schlitzes (106), der sich von einem Ende des Körpers aus einwärts erstreckt, um zwei optisch gekoppelte Szintillatoren (86) zu bilden.

4. Aufbau nach Anspruch 3, bei dem die Lichtverteilung zwischen den zwei Licht-Detektoren, die zum Erfassen von Licht aus den zwei Szintillatoren eines Szintillator-Körpers angeordnet sind, umgekehrt proportional zu der Erstreckung des Schlitzes (106) in den einheitlichen Szintillator-Körper hinein ist.

5. Aufbau nach Anspruch 3 oder 4, bei dem ein reflektierendes Material in dem Schlitz (106) jedes der einheitlichen Szintillator-Körper (76) angeordnet ist.

6. Aufbau nach einem der vorhergehenden Ansprüche, bei dem die Szintillatoren aus Kristallen aus Cäsiumjodid oder thalliumaktiviertem Natriumjodid oder Wismutgermanat-Kristallen oder Kadmiumwolframat gebildet sind und die Licht-Detektoren Photoelektronen-Vervielfacherröhren sind.

7. Aufbau nach einem der vorhergehenden Ansprüche, bei dem die Szintillator-Körper (76, 136) alle in einer Richtung aufeinander in Zeilen und Spanten ausgerichtet angeordnet sind und die Detektoren (78) so angeordnet sind, daß jeder Licht aus zwei Szintillatoren von benachbarten Szintillator-Körpern in jeder von zwei benachbarten Zeilen erfaßt.

8. Aufbau nach einem der Ansprüche 1 bis 6, bei dem die Szintillator-Körper (76, 136) in Seite-an-Seite orientierten Paaren moniert sind, welche Paare in Zeilen und Spalten angeordnet sind, wobei edes Paar rechtwinklig auf die ihm benachbarten Paare in derselben Zeile und in derselben Spalte ausgerichtet ist und die Detektoren in einer Vielzahl von parallelen Zeilen angeordnet sind, wovon jede zum Erfassen von Licht aus einem Szintillator jedes von vier verschiedenen Paaren vorgesehen ist.

9. Radioaktivitätsverteilungs-Erfassungssystem, mit

(a) einem Detektor-Aufbau (62, 54) nach einem der vorhergehenden Ansprüche,

(b) einem Mittel (64) zum Einrichten des Detektor-Aufbaus relativ zu einem zu untersuchenden Patienten (58),

(c) einem Mittel (64) zum Bewirken einer relativen Bewegung zwischen dem Detektor-Aufbau (62, 54) und dem Patienten (58) und

(d) einem Verarbeitungsmittel (20), das wirksam mit dem Detektor-Aufbau (62, 54) verbunden ist und ein Anzeigemittel (22) zum Verarbeiten und Zeigen einer Darstellung der Radioaktivitätsverteilung innerhalb des Patienten (58) enthält.

10. Radioaktivitätsverteilungs-Erfassungssystem nach Anspruch 9, bei dem die relative Bewegung zwischen dem Einrichtmittel und dem Patienten auf einem kreisförmigen Weg um den Patienten herum erfolgt.

11. Radioaktivitätsverteilungs-Erfassungssystem zur Belastungsuntersuchung, mit

(a) einem Übungsmittel (12),

(b) einem Einrichtmittel (14), das dem Übungsmittel (12) wirksam zugeordnet ist, und

(c) einem Strahlungserfassungs-Aufbau (16) nach einem der Ansprüche 1 bis 8, das an dem Einrichtmittel (14) befestigt ist.

12. Radioaktivitätsverteilungs-Erfassungssystem nach Anspruch 11, bei dem das Übungsmittel (12) ein Tretrad ist.

13. Radioaktivitätsverteilungs-Erfassungssystem nach Anspruch 11, bei dem das Übungsmittel ein Übungsfahrrad ist.

14. Radioaktivitätsverteilungs-Erfassungssystem nach Anspruch 11, 12 oder 13, bei dem das Einrichtmittel in drei Dimensionen justierbar ist.

## Revendications

1. Structure de détection de rayonnement, comprenant:

un groupe d'ensembles (76, 136) de scintillateurs, qui fournissent chacun une pluralité de scintillateurs (86) optiquement liés, et

un groupe de détecteurs lumineux (78), qui sont chacun destinés à détecter la lumière venant de quatre scintillateurs (86) différents fournis par quatre ensembles (76, 136) différents de scintillateurs, caractérisée en ce que:

chaque ensemble (76, 136) de scintillateurs ne fournit que deux scintillateurs (86) optiquement liés, et en ce que:

alors que chaque détecteur (78) est destiné à détecter la lumière venant de quatre scintillateurs (86) différentes fournis par quatre ensembles (76, 136) différentes de scintillateurs, la lumière venant des quatre autres scintillateurs (86) appartenant à ces quatre ensembles (76, 136) de scintillateurs est détectée par quatre détecteurs (78) différents.

2. Structure selon la revendication 1, où la lumière venant d'un scintillateur (86), activé par un rayonnement, d'un ensemble (76) de scintillateurs active le détecteur (78) destiné à détecter la lumière venant de ce scintillateur plus que le détecteur destiné à détecter la lumière venant de l'autre scintillateur, optiquement lié, de l'ensemble de scintillateurs.

3. Structure selon la revendication 2, où chaque ensemble (76) de scintillateurs est unitaire, une fente (106) s'étendant vers l'intérieur depuis une extrémité de l'ensemble afin de former les deux scintillateurs (86) optiquement liés.

4. Structure selon la revendication 3, où la distribution de lumière entre les deux détecteurs de lumière destinés à détecter la lumière venant des deux scintillateurs d'un ensemble de scintillateurs est inversement proportionnelle à l'étendue de la fente (106) dans l'ensemble de scintillateurs unitare (76).

5. Structure selon la revendication 3 ou 4, où un matériau réfléchissant est disposé dans la fente (106) de chaque ensemble de scintillateurs unitaire (76).

6. Structure selon l'une quelconque des revendications précédentes, où les scintillateurs sont formés de cristaux d'iodure de césium, ou d'iodure de sodium activé par le thallium, ou de cristaux de germanante de bismuth, ou de tungstate de cadmium, et les détecteurs lumineux sont des tubes photo-multiplicateurs.

7. Structure selon l'une quelconque des revendications précédentes, où les ensembles (76, 136) de scintillateurs sont disposés, tous étant alignés suivant une seule direction, en des rangées et des colonnes, et les détecteurs (78) sont disposés de façon à détecter chacun la lumière venant de deux scintillateurs, d'ensembles voisins de scintillateurs, dans chacune de deux rangées adjacentes.

8. Structure selon l'une quelconque des revendications 1 à 6, où les ensembles (76, 136) de scintillateurs sont montés en paires placées côté à côte, les paires étant disposées en rangées et en colonnes de façon que chaque paire soit alignée perpendiculairement aux paires qui lui sont voisines dans la même rangée et dans la même colone, et les détecteurs sont disposés en plusieurs rangées parallèles afin de détecter chaun la lumière venant d'un seul scintillateur de chacune des quatre pairs différentes.

9. Système de détection d'une distribution de radioactivité comprenant:

(a) une structure détectrice (62, 54) selon l'une quelconque des revendications précédentes;

(b) un moyen (64) servant au montage de la structure détectrice relativement à un sujet (58) analysé;

(c) un moyen (64) servant à effectuer un déplacement relatif entre la structure détectrice (62, 54) et le sujet (58); et

(d) un moyen de traitement (20) fonctionnellement couplé à ladite structure détectrice (62, 54) et comportant un moyen d'affichage (22), lequel moyen de traitement sert à traiter et présenter une représentation de la distribution de radioacti-

7

vité à l'intérieur du sujet (58).

10. Système de détection d'une distribution de radioactivité selon la revendication 9, où le déplacement relatif entre le moyen de montage et le sujet a lieu selon un trajet circulaire autour du sujet.

11. Système de détection d'une distribution de radioactivité pour l'analyse de l'état de tension d'un sujet, comprenant:

(a) un moyen d'exercice (12);

(b) un moyen de montage (14) fonctionnellement associé audit moyen d'exercice (12); et

(c) une structure de détection de rayonnement (16) selon l'une quelconque des revendications 1 à 8, fixée audit moyen de montage (14).

12. Système de détection d'une distribution de radioactivité selon la revendication 11, où ledit moyen d'exercice (12) est un moulin de discipline, ou roue à marches.

13. Système de détection d'une distribution de radioactivité selon la revendication 11, où ledit moyen d'exercice (12) est un vélo d'appartement, ou pédaleur.

14. Système de détection d'une distribution de radioactivité selon la revendication 11, 12 ou 13, où ledit moyen de montage (14) est ajustable dans les trois dimensions.

Fig. 1

Fig. 2

*Fig. 3*

*Fig. 4*

Fig. 5-I

FIG. 5 - II

Fig. 6

Fig. 7

Fig. 8

*Fig. 9*

*Fig. 10*

Fig. 11

Fig. 12

Fig. 13